# EUROPEAN PATENT SPECIFICATION

(11) **EP 3 230 589 B1**
(45) Date of publication and mention of the grant of the patent: **17.06.2020**
(21) Application number: 15817510.9
(22) Date of filing: 10.12.2015
(51) Int. Cl.: F04B 43/12

(54) **PERISTALTIC PUMPS**
PERISTALTISCHE PUMPEN
POMPES PÉRISTALTIQUES

(30) Priority: 10.12.2014 GB 201421964
(43) Date of publication of application: 18.10.2017
(73) Proprietor: Hodges&Drake Design Limited, Leicester LE1 5TT (GB)
(72) Inventor: HODGES, Kevin, Leicester Leicestershire LE1 5TT (GB)
(74) Representative: Serjeants LLP
(86) International application number: PCT/GB2015/053777
(87) International publication number: WO 2016/092307

(56) References cited:
- WO-A1-94/10446
- WO-A2-88/05868
- DE-A1- 3 320 091
- DE-U1- 8 316 229
- US-A- 4 138 205
- US-A- 4 576 556
- US-A1- 2011 058 969

## Description

### Technical Field

The present disclosure relates generally to peristaltic pumps.

### Technical Background

Peristaltic pumps are used to pump liquids in a wide variety of applications, in particular where the flow of liquid needs to be carefully metered and where contamination of the liquid needs to be avoided. They are extensively used in medical applications, for example to deliver intravenous (IV) liquids to a patient, and also in food and beverage applications, for example to dispense a predetermined quantity of a beverage or a component of a beverage such as a liquid flavouring.

In a conventional peristaltic pump, flexible tubing is compressed between the pressing members (e.g. pins or rollers) of a rotor and a stator, and liquid is conveyed through the flexible tubing as the rotor rotates. The friction between the pressing members and the tubing can, however, cause a number of problems, notably premature wear of the flexible tubing, and the present disclosure seeks to address this.

The preamble of claim 1 is derivable from DE 3320091 A1 and WO 88/05868 A2.

### Summary of the Disclosure

According to a first aspect of the present disclosure, there is provided a peristaltic pump comprising:
a drivable rotor having at least one pressing member;
a cylindrical stator in which the rotor is rotatable;
flexible tubing having an inlet side and an outlet side, the flexible tubing extending circumferentially around the stator against an inner wall;
a radially deformable ring positioned between the rotor and the circumferentially extending flexible tubing, the ring being deformable by the at least one pressing member upon rotation of the rotor to compress the flexible tubing against the inner wall of the cylindrical stator to thereby convey liquid along the flexible tubing;
wherein the radially deformable ring includes a ring anchor;
wherein the inlet side and the outlet side of the flexible tubing are arranged side-by-side so that the flexible tubing extends in a substantially radial direction outwardly away from the cylindrical stator;
characterized in that the ring anchor projects radially outwardly from the radially deformable ring and is located between, and gripped by, the inlet side and the outlet side of the flexible tubing to prevent rotation of the radially deformable ring during rotation of the rotor.

According to a second aspect of the present disclosure, there is provided a method for assembling a peristaltic pump according to the first aspect, the method comprising:
locating the flexible tubing circumferentially around the radially deformable ring and in contact therewith, with the inlet side and the outlet side of the flexible tubing arranged side-by-side on either side of the ring anchor;
positioning the flexible tubing and the radially deformable ring in the cylindrical stator with the flexible tubing arranged against the inner wall of the cylindrical stator; and
fitting the rotor in the cylindrical stator by simultaneously rotating the rotor and pressing the rotor into the centre of the radially deformable ring.

The term 'liquid' as used in this specification is intended to include liquid and semiliquid products.

The rotor may include a plurality of pressing members and the pressing members may be equispaced around the circumference of the rotor. The rotor may include a spindle. The spindle and the or each pressing member may be integrally formed. The or each pressing member may be a lobe.

In one embodiment, the or each lobe may have an arcuate pressing surface which may be arranged to progressively compress the flexible tubing against the inner wall of the cylindrical stator during rotation of the rotor. The or each lobe may have an apex at which the arcuate pressing surface terminates, and the apex may be arranged to fully compress the flexible tubing against the inner wall of the cylindrical stator. The rotor may include two of said lobes at diametrically opposite locations.

In a conventional peristaltic pump, the flexible tubing is subjected to high rates of wear because of the friction forces applied by the pressing members during rotation of the rotor. It is, therefore, generally necessary to use expensive high-grade flexible tubing that can withstand the high friction forces to avoid premature wear of the flexible tubing. In the peristaltic pump according to the present disclosure, the radially deformable ring prevents direct contact between the pressing members and the flexible tubing, the radial compression force instead being applied to the flexible tubing by the radially deformable ring. As a result, the flexible tubing does not wear out during operation of the pump. In addition, the flexible tubing is not stretched or pinched because the radially deformable ring is held stationary by the ring anchor. This means that lower grade (and, therefore, less expensive) flexible tubing can typically be used.

The rotor may be engageable by an external rotary drive. With this arrangement, the peristaltic pump is easy and cheap to manufacture and can be readily provided as a disposable system. In particular, because the rotary drive is a separate component that engages the rotor of the peristaltic pump, the peristaltic pump has a simple and inexpensive construction which can, for example, be formed integrally with or attached to a liquid container and which can be disposed of with the liquid container, for example when the container is empty.

The ring anchor may comprise a finger projecting radially outwardly from the radially deformable ring.

The radially deformable ring may have an axial depth which is greater than an outer diameter of the flexible tubing.

The radially deformable ring may include a plurality of circumferentially-spaced radial projections on a first axial rim which may project in a radially outward direction towards the inner wall of the cylindrical stator. The radial projections may help to axially retain the flexible tubing on the radially deformable ring, in particular whilst the flexible tubing and the radially deformable ring are being positioned in the cylindrical stator during assembly of the peristaltic pump.

The rotor may include a circular flange which may axially retain the flexible tubing and the radially deformable ring in the cylindrical stator. In an embodiment, the radially deformable ring may be arranged in the stator with the radial projections in contact with the circular flange. In this embodiment, the radial projections act as plain bearing members and space the flexible tubing from the axially inner surface of the rotating circular flange. This reduces friction forces between the rotating circular flange and the static flexible tubing as the rotor rotates and prevents the flexible tubing from being gripped and stretched by the circular flange during rotation of the rotor.

The radially deformable ring may include a locating arrangement. The locating arrangement may be provided on a second axial rim. The locating arrangement may extend from the ring anchor over the inlet side and the outlet side of the flexible tubing. The locating arrangement may comprise a locating flange or may alternatively comprise a pair of oppositely extending locating projections.

The radially deformable ring may include locating members which may provide for axial location of the flexible tubing on the radially deformable ring. The locating members may be provided on first and second rims at axially opposite ends of the radially deformable ring. The locating members ensure that the flexible tubing is retained axially on the radially deformable ring, in particular whilst the flexible tubing and radially deformable ring are being positioned in the cylindrical stator during assembly of the peristaltic pump.

The locating members may include a plurality of circumferentially-spaced locating projections, which may project in a radially outward direction, on the first rim. The locating projections may be equally spaced around the first rim. The locating members may include a locating flange, on the second rim, which extends from the ring anchor over the inlet side and the outlet side of the flexible tubing.

### Brief Description of the Drawings

Figures 1a and 1b are cross-sectional views of a peristaltic pump according to a first embodiment of the present disclosure with the rotor at different rotational positions;
Figures 2a and 2b are perspective views showing the detail of the radially deformable ring shown in Figures 1a and 1b;
Figure 3 is a perspective view showing the flexible tubing located around the radially deformable ring before being positioned in the cylindrical stator;
Figure 4 is a perspective view showing the flexible tubing and radially deformable ring positioned in the cylindrical stator before the rotor is fitted in the cylindrical stator;
Figure 5 is a detailed view of the rotor;
Figures 6a and 6b are cross-sectional views of a peristaltic pump according to a second embodiment of the present disclosure with the rotor at different rotational positions;
Figures 7a and 7b are perspective views showing the detail of the radially deformable ring shown in Figures 6a and 6b;
Figure 8 is an axial view of the radially deformable ring of Figures 7a and 7b with the flexible tubing located around the radially deformable ring;
Figure 9 is a perspective view showing the flexible tubing located around the radially deformable ring before being positioned in the cylindrical stator;
Figure 10 is a perspective view showing the flexible tubing and radially deformable ring positioned in the cylindrical stator before the rotor is fitted in the cylindrical stator;
Figure 11 is a detailed view of the rotor; and
Figures 12a and 12b are cross-sectional views of the second embodiment with the rotor at different rotational positions.

### Detailed Description of Embodiments

Embodiments of the present disclosure will now be described by way of example only and with reference to the accompanying drawings.

A peristaltic pump 10, 50 includes a cylindrical stator 12. Although not shown, the cylindrical stator 12 can be integrally formed, for example as a one-piece moulding, with a liquid container from which liquid is to be dispensed or can be removably mountable on a liquid container.

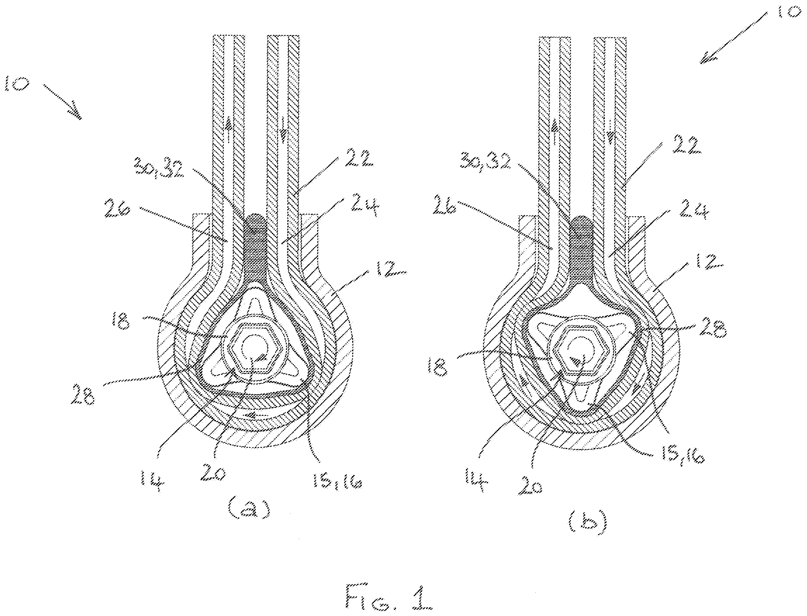

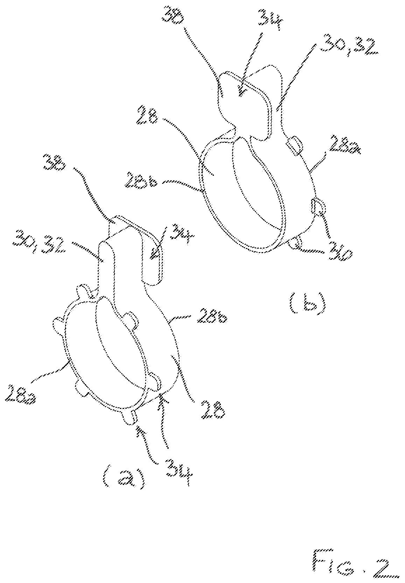

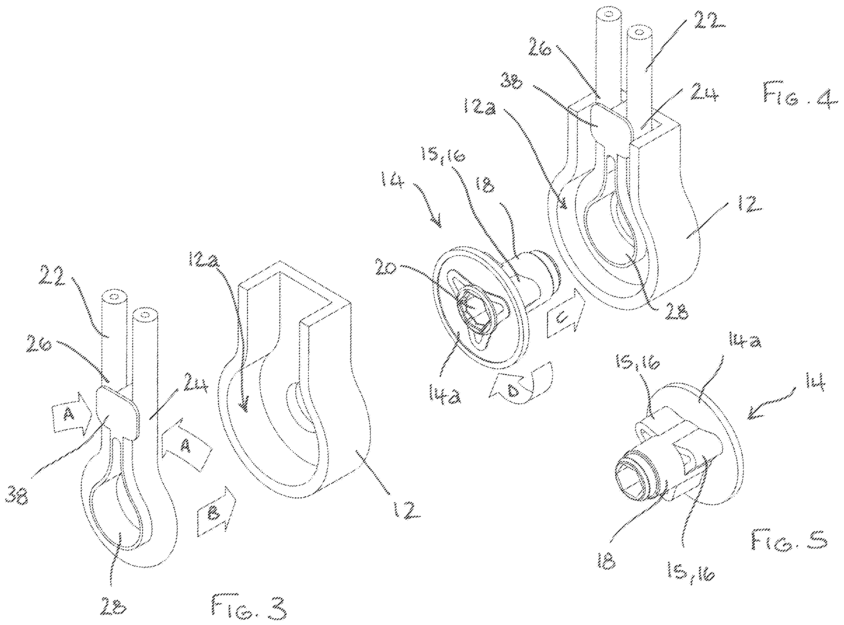

Figures 1 to 5 illustrate a first embodiment of a peristaltic pump 10 which includes a rotor 14 (best seen in Figure 5), typically formed of a moulded substantially rigid plastics material. The rotor 14 includes a plurality of pressing members 15 in the form of lobes 16 which are integrally formed with, and project radially outwardly from, a spindle 18 and which are equally spaced around the circumference of the spindle 18. In the illustrated embodiment, the rotor 14 includes three lobes 16 but it will be appreciated that the rotor 14 can include any suitable number of lobes 16. The spindle 18 includes a central drive aperture 20 which can be engaged by an external rotary drive (not shown) such as the drive shaft of an electric motor.

The peristaltic pump 10 includes flexible tubing 22 which can be formed of any suitable resilient plastics material such as polyvinyl chloride. The flexible tubing 22 has an inlet side 24 through which liquid is delivered to the peristaltic pump 10 and an outlet side 26 through which liquid is delivered from the peristaltic pump 10. The inlet side 24 and outlet side 26 are designated with respect to the normal direction of rotation of the rotor 14 (clockwise in the accompanying drawings). The inlet side 24 is typically connected to a liquid outlet port of a liquid container (not shown) and the outlet side 26 is arranged to deliver the liquid to a desired location. The flexible tubing 22 extends circumferentially around the cylindrical stator 12 against an inner wall 12a and the inlet side 24 and the outlet side 26 are arranged side-by-side, at circumferentially adjacent positions around the cylindrical stator 12. The inlet side 24 and the outlet side 26 extend outwardly away from the cylindrical stator 12 in a substantially radial direction.

A radially deformable ring 28, comprising a suitable resiliently deformable material (typically a plastics material), is positioned between the rotor 14 and the circumferentially extending flexible tubing 22. The deformable ring 28 is contacted by the lobes 16 of the rotor 14 as best seen in Figures 1a and 1b and is deformed radially outwardly by the lobes 16. The radially outward deformation of the deformable ring 28 compresses the flexible tubing 22 against the inner wall 12a of the cylindrical stator 12 and, thus, as the rotor 14 is rotated by an external rotary drive, the compression of the flexible tubing 22 between the deformable ring 28 and the inner wall 12a conveys liquid along the flexible tubing 22 by peristaltic action, between the inlet side 24 and the outlet side 26. Although the liquid is normally conveyed from the inlet side 24 to the outlet side 26 of the flexible tubing (by rotating the rotor 14 in the clockwise direction as viewed in Figures 1a and 1b), the flow direction can be easily reversed and it will be understood that the desired flow direction can be selected by simply selecting a clockwise or anti-clockwise direction of rotation for the external rotary drive.

The deformable ring 28 includes a ring anchor 30 in the form of a finger 32 which projects radially from the deformable ring 22. The ring anchor 30 is located between the inlet side 24 and the outlet side 26 of the flexible tubing 22 and has a sufficient length (in the radially outward direction) and width (in the circumferential direction) that it cannot move out of its position between the inlet side 24 and the outlet side 26 of the flexible tubing 22. Thus, it will be understood that the ring anchor 30 prevents the deformable ring 28 from being rotated by the rotor 14 as the rotor 14 rotates in the cylindrical stator 12. If the ring anchor 30 was not provided, the deformable ring 28 would be caused to rotate by the rotor 14 and this would result in unwanted stretching and wearing of the flexible tubing 22 between the deformable ring 28 and the inner wall 12a of the cylindrical stator 12.

As best seen in Figure 3, the deformable ring 28 has an axial depth which is greater than the outer diameter of the flexible tubing 22. In order to assist with the assembly of the peristaltic pump 10, the deformable ring 28 includes a plurality of locating members 34 which help to locate the flexible tubing 22 around the deformable ring 28 and prevent the flexible tubing 22 from slipping axially off the deformable ring 28.

In the illustrated embodiment, the locating members 34 comprise a plurality of locating projections 36 on a first rim 28a of the deformable ring 28. The locating projections 36 project radially outwardly by a small distance from the deformable ring 28, in use towards the inner wall 12a of the cylindrical stator 12, and are typically provided at equispaced positions around the circumference of the first rim 28a. The locating members 34 additionally comprise a locating flange 38, on the second rim 28b, which extends sideways from the ring anchor 30 over the inlet side 24 and the outlet side 26 of the flexible tubing 22. Thus, it will be understood that the locating projections 36 prevent the flexible tubing 22 from slipping axially of the first rim 28a of the deformable ring 22 and that the locating flange 38 helps to prevent the flexible tubing 22 from slipping axially off the second rim 28b, in the opposite direction.

The method of assembling the peristaltic pump 10 will now be described with reference to Figures 3 and 4. Initially, the flexible tubing 22 is located around the deformable ring 28 so that it contacts the radially outer surface of the deformable ring 28 and so that the inlet side 24 and the outlet side 26 of the flexible tubing 22 are arranged side-by-side on either side of the ring anchor 30. As will be understood, the locating projections 36 and locating flange 38 help the user to locate the flexible tubing 22 around the deformable ring 28 and prevent the flexible tubing 22 from slipping axially off the deformable ring 28 during assembly. The assembled flexible tubing 22 and deformable ring 28 are then compressed, for example by squeezing as shown diagrammatically by the arrows A, to a sufficient size to enable them to be pushed into the cylindrical stator 12 in the direction of the arrow B.

Once the flexible tubing 22 and deformable ring 28 have been positioned in the cylindrical stator 12 as shown in Figure 4, the rotor 14 can be fitted and this is achieved by pushing the rotor 14 into the centre of the deformable ring 28, as shown diagrammatically by the arrow C, and at the same time rotating the rotor 14 by a small amount as shown diagrammatically by the arrow D. Once the rotor 14 has been fitted into the centre of the deformable ring 28, the circular flange 14a may also help to retain the flexible tubing 22 in the correct position inside the cylindrical stator 12. Once assembled, the central drive aperture 20 can be engaged by an external rotary drive which can be operated to rotate the rotor 14.

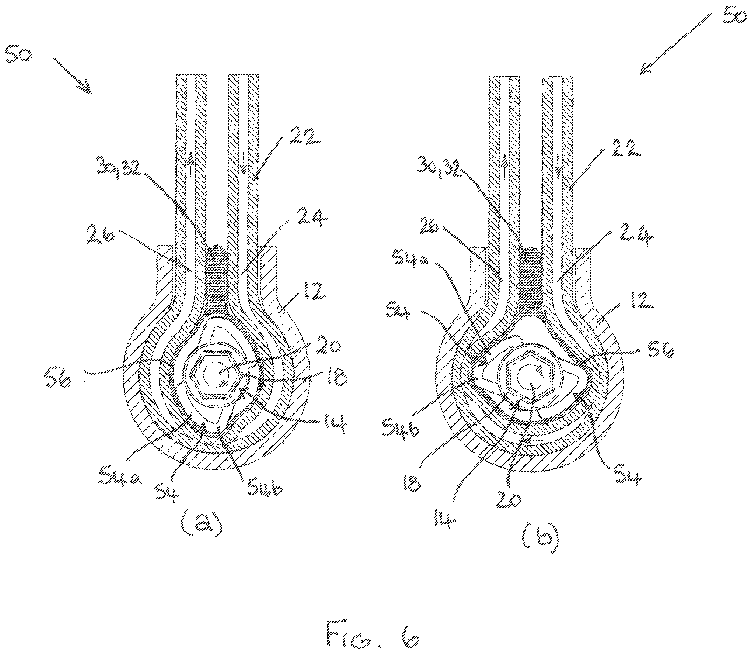

Referring now to Figures 6 to 12, there is shown a second embodiment of a peristaltic pump 50. The peristaltic pump 50 shares many features in common with the peristaltic pump 10 illustrated in Figures 1 to 5 and corresponding features are designated using corresponding reference numerals. The differences between the peristaltic pumps 10, 50 will now be explained.

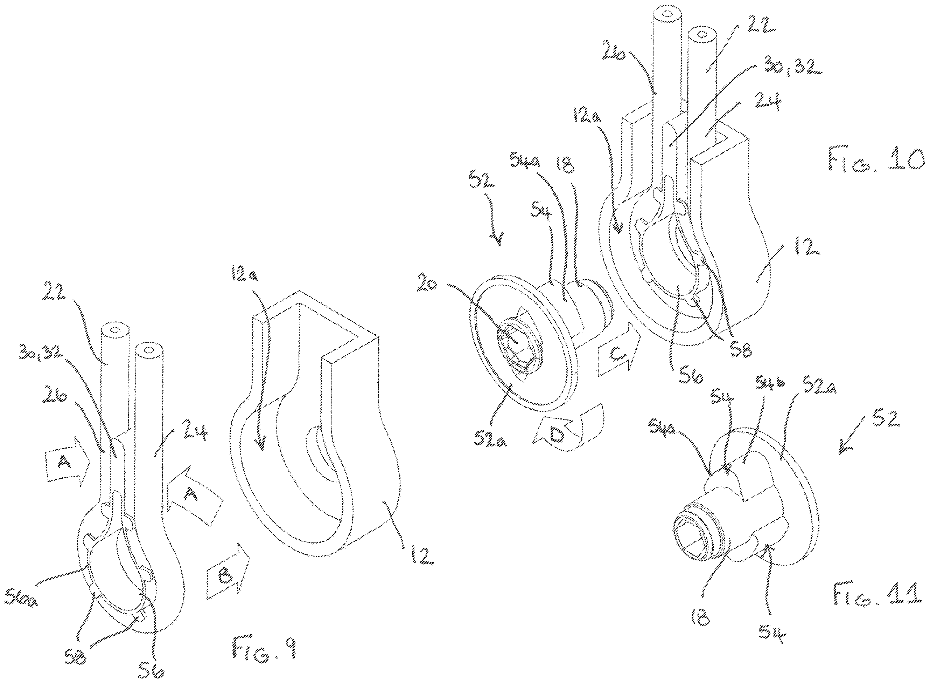

The peristaltic pump 50 includes a rotor 52 (best seen in Figure 11) which includes two diametrically opposed lobes 54 that are integrally formed with, and project radially outwardly from, the spindle 18. As best seen in Figures 6a and 6b, each lobe 54 has a curved or arcuate pressing surface 54a whose radius relative to the spindle axis increases gradually and smoothly. The pressing surface 54a progressively compresses the flexible tubing 22 against the inner wall 12a of the cylindrical stator 12 as the rotor 52 rotates in the cylindrical stator 12 in the clockwise direction. Each lobe 54 also has an apex 54b at which the pressing surface 54a terminates and it will be apparent from Figures 6a and 6b that each apex 54b is arranged to fully compress the flexible tubing 22 against the inner wall 12a of the cylindrical stator 12 to achieve the required peristaltic pumping action.

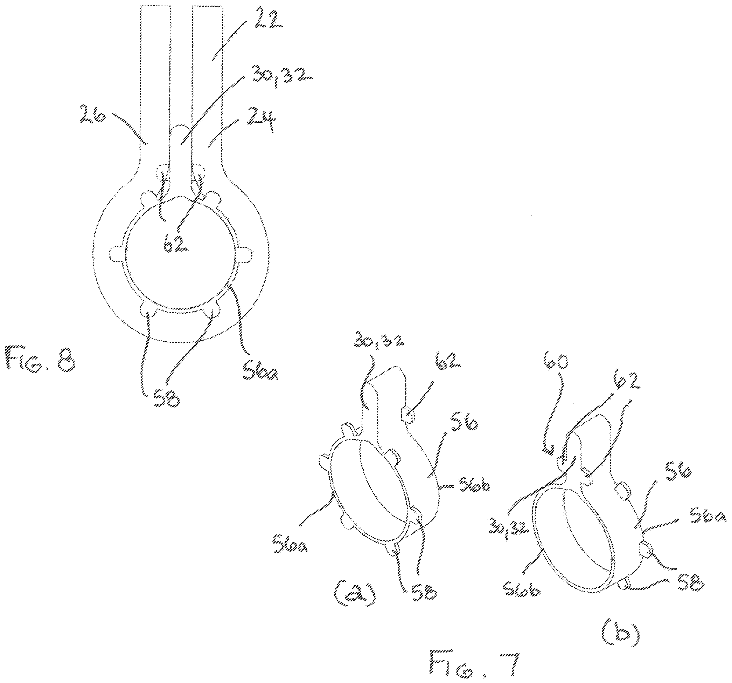

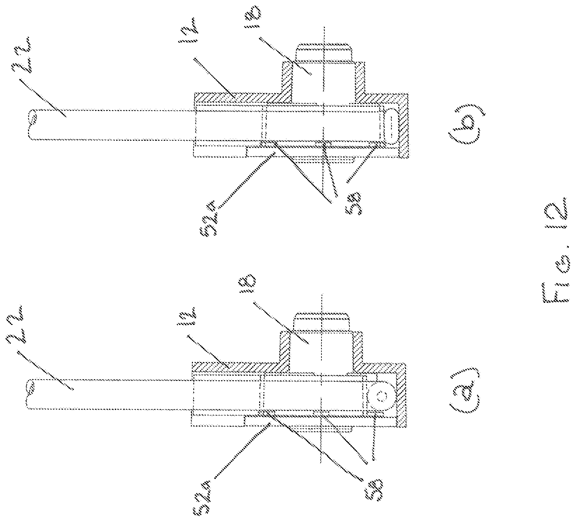

Referring in particular to Figures 7 and 8, the peristaltic pump 50 comprises a radially deformable ring 56 having a plurality of circumferentially-spaced radial projections 58 on a first axial rim 56a. The radial projections 58 project in a radially outward direction in use towards the inner wall 12a of the cylindrical stator 12. The rotor 52 includes a circular flange 52a which axially retains the flexible tubing 22 and the radially deformable ring 56 in the cylindrical stator 12. As best seen in Figures 12a and 12b, the radially deformable ring 56 is arranged in the cylindrical stator 12 of the peristaltic pump 50 with the radial projections 58 in contact with the circular flange 52a. The radial projections 58 act as plain bearing members or bearing flanges and space the flexible tubing 22 from the axially inner surface of the circular flange 52a. As noted above, this reduces or eliminates friction forces between the rotating circular flange 52a and the flexible tubing 22 as the rotor 52 rotates and prevents the flexible tubing 22 from being gripped and stretched by the circular flange 52a.

Although in the second embodiment the primary function of the radial projections 58 is to act as bearing members or bearing flanges, it will also be understood that the radial projections 58 assist with axial location and retention of the flexible tubing 22 on the radially deformable ring 56 in the same way as the locating projections 36 of the first embodiment.

In order to further assist with axial location and retention of the flexible tubing 22, the radially deformable ring 56 can optionally include a locating arrangement 60 on the second axial rim 56b (best seen in Figures 7 and 8). The locating arrangement 60 comprises a pair of oppositely extending locating projections 62 which extend from the ring anchor 30 over the inlet side 24 and the outlet side 26 of the flexible tubing 22.

The method of assembling the peristaltic pump 50 is essentially the same as the assembly method described above with reference to Figures 3 and 4. That is, the flexible tubing 22 is initially located around the deformable ring 56 as shown in Figure 9 so that it contacts the radially outer surface of the deformable ring 56 and so that the inlet side 24 and the outlet side 26 of the flexible tubing 22 are arranged side-by-side on either side of the ring anchor 30. In this embodiment, the radial projections 58 and the locating projections 62 may help the user to locate the flexible tubing 22 around the deformable ring 56 and may help to prevent the flexible tubing 22 from slipping axially off the deformable ring 56 during assembly. The assembled flexible tubing 22 and deformable ring 56 are then compressed, for example by squeezing as shown diagrammatically by the arrows A, to a sufficient size to enable them to be pushed into the cylindrical stator 12 in the direction of the arrow B with the radial projections 58 uppermost.

Once the flexible tubing 22 and deformable ring 56 have been positioned in the cylindrical stator 12 as shown in Figure 10, the rotor 52 can be fitted and this is achieved by pushing the rotor 14 into the centre of the deformable ring 56, as shown diagrammatically by the arrow C, and at the same time rotating the rotor 52 by a small amount as shown diagrammatically by the arrow D. Once the rotor 52 has been fitted into the centre of the deformable ring 56, the circular flange 52a contacts the radial projections 58 which, as explained above, act as bearing flanges which space the flexible tubing 22 from the circular flange 52a. Once assembled, the central drive aperture 20 can be engaged by an external rotary drive which can be operated to rotate the rotor 52.

Although exemplary embodiments have been described in the preceding paragraphs, it should be understood that various modifications may be made to those embodiments without departing from the scope of the appended claims. Thus, the breadth and scope of the claims should not be limited to the above-described exemplary embodiments. Each feature disclosed in the specification, including the claims and drawings, may be replaced by alternative features serving the same, equivalent or similar purposes, unless expressly stated otherwise.

For example, the rotor 14, 52 could include a projection in place of the central drive aperture 20 which could engage an aperture in a drive shaft of an external rotary drive.

Unless the context clearly requires otherwise, throughout the description and the claims, the words "comprise", "comprising", and the like, are to be construed in an inclusive as opposed to an exclusive or exhaustive sense; that is to say, in the sense of "including, but not limited to".

Any combination of the above-described features in all possible variations thereof is encompassed by the present invention unless otherwise indicated herein or otherwise clearly contradicted by context.

## Claims

1. A peristaltic pump (10;50) comprising:
a drivable rotor (14;52) having at least one pressing member (15);
a cylindrical stator (12) in which the drivable rotor (10;50) is rotatable;
flexible tubing (22) having an inlet side (24) and an outlet side (26), the flexible tubing (22) extending circumferentially around the stator (12) against an inner wall (12a);
a radially deformable ring (28;56) positioned between the rotor (14, 52) and the circumferentially extending flexible tubing (22), the radially deformable ring (28;56) being deformable by the at least one pressing member (15) upon rotation of the rotor (14, 52) to compress the flexible tubing (22) against the inner wall (12a) of the cylindrical stator (12) to thereby convey liquid along the flexible tubing (22);
wherein the radially deformable ring (28;56) includes a ring anchor (30);
wherein the inlet side (24) and the outlet side (26) of the flexible tubing (22) are arranged side-by-side so that the flexible tubing (22) extends in a substantially radial direction outwardly away from the cylindrical stator (12);
**characterized in that** the ring anchor (30) projects radially outwardly from the radially deformable ring (28;56) and is located between, and gripped by, the inlet side (24) and the outlet side (26) of the flexible tubing (22) to prevent rotation of the radially deformable ring (28;56) during rotation of the drivable rotor (14; 52).

2. A pump according to claim 1, wherein the ring anchor (30) comprises a finger (32) projecting radially outwardly from the radially deformable ring (28, 56).

3. A pump according to claim 1 or claim 2, wherein the radially deformable ring (28, 56) has an axial depth which is greater than an outer diameter of the flexible tubing (22).

4. A pump according to any preceding claim, wherein the radially deformable ring (28, 56) includes a plurality of circumferentially-spaced radial projections (36, 58) on a first axial rim (28a, 56a) which project in a radially outward direction towards the inner wall (12a) of the cylindrical stator (12).

5. A pump according to claim 4, wherein the rotor (14, 52) includes a circular flange (14a, 52a) which axially retains the flexible tubing (22) and the radially deformable ring (28, 56) in the cylindrical stator (12) and wherein the radially deformable ring (56) is arranged in the stator (12) with the radial projections (58) in contact with the circular flange (52a).

6. A pump according to any preceding claim, wherein the radially deformable ring (28, 56) includes a locating arrangement (34, 60), on a second axial rim (28b, 56b), which extends from the ring anchor (30) over the inlet side (24) and the outlet side (26) of the flexible tubing (22).

7. A pump according to claim 6, wherein the locating arrangement (34, 60) comprises a locating flange (38) or a pair of oppositely extending locating projections (62).

8. A pump according to any preceding claim, wherein the rotor (14, 52) includes a plurality of pressing members (15) which are equispaced around the circumference of the rotor (14, 52).

9. A pump according to any preceding claim, wherein the rotor (14, 52) includes a spindle (18) and the or each pressing member (15) is integrally formed with the spindle (18).

10. A pump according to any preceding claim, wherein the or each pressing member (15) is a lobe (16, 54).

11. A pump according to claim 10, wherein the or each lobe (54) has an arcuate pressing surface (54a) which is arranged to progressively compress the flexible tubing (22) against the inner wall (12a) of the cylindrical stator (12) during rotation of the rotor (52).

12. A pump according to claim 11, wherein the or each lobe (54) has an apex (54b) at which the arcuate pressing surface (54a) terminates, the apex (54b) being arranged to fully compress the flexible tubing (22) against the inner wall (12a) of the cylindrical stator (12).

13. A pump according to claim 11 or claim 12, wherein the rotor (52) includes two of said lobes (54) at diametrically opposite locations.

14. A pump according to any preceding claim, wherein the rotor (14, 52) is engageable by an external rotary drive.

15. A method for assembling a peristaltic pump (10; 50) according to any preceding claim, the method comprising:
locating the flexible tubing (22) circumferentially around the radially deformable ring (28; 56) and in contact therewith, with the inlet side (24) and the outlet side (26) of the flexible tubing (22) arranged side-by-side on either side of the ring anchor (30); whereby the ring anchor (30) is gripped by the inlet side (24) and the outlet side (26) of the flexible tubing (22) to prevent rotation of the radially deformable ring (28; 56) during rotation of the rotor (14; 52),
positioning the flexible tubing (22) and the radially deformable ring (28;56) in the cylindrical stator (12) with the flexible tubing (22) arranged against the inner wall (12a) of the cylindrical stator (12); and
fitting the rotor (14;52) in the cylindrical stator (12) by simultaneously rotating the rotor (14, 52) and pressing the rotor (14;52) into the centre of the radially deformable ring (28;56).

## Patentansprüche

1. Peristaltische Pumpe (10; 50), die Folgendes umfasst:
einen antreibbaren Rotor (14; 52), der mindestens ein Druckelement (15) aufweist;
einen zylindrischen Stator (12), in dem der drehbare Rotor (10; 50) drehbar ist;
eine biegsame Verrohrung (22), die eine Einlassseite (24) und eine Auslassseite (26) aufweist, wobei sich die biegsame Verrohrung (22) umfänglich um den Stator (12) gegen eine Innenwand (12a) erstreckt;
einen radial verformbaren Ring (28; 56), der zwischen dem Rotor (14, 52) und der sich umfänglich erstreckenden biegsamen Verrohrung (22) positioniert ist, wobei der radial verformbare Ring (28; 56) durch das mindestens eine Druckelement (15) bei Drehung des Rotors (14, 52) verformbar ist, um die biegsame Verrohrung (22) gegen die Innenwand (12a) des zylindrischen Stators (12) zu komprimieren, wodurch Flüssigkeit entlang der biegsamen Verrohrung (22) befördert wird;
wobei der radial verformbare Ring (28; 56) einen Ringanker (30) beinhaltet;
wobei die Einlassseite (24) und die Auslassseite (26) der biegsamen Verrohrung (22) Seite an Seite derart eingerichtet sind, dass sich die biegsame Verrohrung (22) in eine im Wesentlichen radiale Richtung auswärts von dem zylindrischen Stator (12) weg erstreckt;
**dadurch gekennzeichnet, dass** der Ringanker (30) radial auswärts von dem radial verformbaren Ring (28; 56) vorragt und zwischen der Einlassseite (24) und der Auslassseite (26) der biegsamen Verrohrung (22) liegt und davon erfasst ist, um Drehung des sich radial verformbaren Rings (28; 56) während der Drehung des antreibbaren Rotors (14; 52) zu verhindern.

2. Pumpe nach Anspruch 1, wobei der Ringanker (30) einen Finger (32) umfasst, der radial von dem radial verformbaren Ring (28, 56) vorragt.

3. Pumpe nach Anspruch 1 oder 2, wobei der radial verformbare Ring (28, 56) eine axiale Tiefe aufweist, die größer ist als ein Außendurchmesser der biegsamen Verrohrung (22).

4. Pumpe nach einem vorstehenden Anspruch, wobei der radial verformbare Ring (28, 56) eine Vielzahl umfänglich beabstandeter radialer Vorsprünge (36, 58) auf einem ersten axialen Rand (28a, 56a) beinhaltet, die in eine radiale Auswärtsrichtung zu der Innenwand (12a) des zylindrischen Stators (12) vorragen.

5. Pumpe nach Anspruch 4, wobei der Rotor (14, 52) einen kreisförmigen Flansch (14a, 52a) beinhaltet, der axial die biegsame Verrohrung (22) und den radial verformbaren Ring (28, 56) in dem zylindrischen Stator (12) hält, und wobei der radial verformbare Ring (56) in dem Stator (12) mit den radialen Vorsprüngen (58) in Kontakt mit dem kreisförmigen Flansch (52a) eingerichtet ist.

6. Pumpe nach einem vorstehenden Anspruch, wobei der radial verformbare Ring (28, 56) eine Lokalisierungseinrichtung (34, 60) auf einem zweiten axialen Rand (28b, 56b) beinhaltet, die sich von dem Ringanker (30) über die Einlassseite (24) und die Auslassseite (26) der biegsamen Verrohrung (22) erstreckt.

7. Pumpe nach Anspruch 6, wobei die Lokalisierungseinrichtung (34, 60) einen Lokalisierungsflansch (38) oder ein Paar sich entgegengesetzt erstreckender Lokalisierungsvorsprünge (62) umfasst.

8. Pumpe nach einem vorstehenden Anspruch, wobei der Rotor (14, 52) eine Vielzahl von Druckelementen (15) beinhaltet, die um den Umfang des Rotors (14, 52) regelmäßig beabstandet sind.

9. Pumpe nach einem vorstehenden Anspruch, wobei der Rotor (14, 52) eine Spindel (18) beinhaltet, und das oder jedes Druckelement (15) integral mit der Spindel (18) gebildet ist.

10. Pumpe nach einem vorstehenden Anspruch, wobei das oder jedes Druckelement (15) ein Kolben (16, 54) ist.

11. Pumpe nach Anspruch 10, wobei der oder jeder Kolben (54) eine bogenförmige Druckoberfläche (54a) aufweist, die eingerichtet ist, um die biegsame Verrohrung (22) allmählich gegen die Innenwand (12a) des zylindrischen Stators (12) während der Drehung des Rotors (52) zu komprimieren.

12. Pumpe nach Anspruch 11, wobei der oder jeder Kolben (54) einen Scheitelpunkt (54b) aufweist, an dem die bogenförmige Druckoberfläche (54a) endet, wobei der Scheitelpunkt (54b) eingerichtet ist, um die biegsame Verrohrung (22) vollständig gegen die Innenwand (12a) des zylindrischen Stators zu komprimieren.

13. Pumpe nach Anspruch 11 oder 12, wobei der Rotor (52) zwei der Kolben (54) an diametral entgegengesetzten Stellen beinhaltet.

14. Pumpe nach einem vorstehenden Anspruch, wobei der Rotor (14, 52) von einem externen Drehantrieb einrückbar ist.

15. Verfahren zum Zusammenfügen einer peristaltischen Pumpe (10; 50) nach einem vorstehenden Anspruch, wobei das Verfahren Folgendes umfasst:
Lokalisieren der biegsamen Verrohrung (22) umfänglich um den radial verformbaren Ring (28; 56) und in Kontakt damit, wobei die Einlassseite (24) und die Auslassseite (26) der biegsamen Verrohrung (22) Seite an Seite auf jeder Seite des Ringankers (30) angeordnet sind; wodurch der Ringanker (30) von der Einlassseite (24) und der Auslassseite (26) der biegsamen Verrohrung (22) erfasst wird, um Drehung des radial verformbaren Rings (28; 56) während der Drehung des Rotors (14; 52) zu verhindern,
Positionieren der biegsamen Verrohrung (22) und des radial verformbaren Rings (28; 56) in dem zylindrischen Stator (12) mit der biegsamen Verrohrung (22) gegen die Innenwand (12a) des zylindrischen Stators (12) eingerichtet; und
Einpassen des Rotors (14; 52) in den zylindrischen Stator (12) durch gleichzeitiges Drehen des Rotors (14, 52) und Drücken des Rotors (14; 52) in die Mitte des radial verformbaren Rings (28; 56).

## Revendications

1. Pompe péristaltique (10 ; 50) comprenant :
un rotor pouvant être entraîné (14; 52) ayant au moins un élément de pression (15) ;
un stator cylindrique (12) dans lequel le rotor pouvant être entraîné (14 ; 52) peut tourner;
un tubage flexible (22) ayant un côté entrée (24) et un côté sortie (26), le tubage flexible (22) s'étendant de manière circonférentielle autour du stator (12) contre une paroi interne (12a) ;
une bague pouvant se déformer radialement (28 ; 56) positionnée entre le rotor (14, 52) et le tubage flexible s'étendant de manière circonférentielle (22), la bague pouvant se déformer radialement (28; 56) étant déformable par le ou les éléments de pression (22) lors de la rotation du rotor (14, 52) pour comprimer le tubage flexible (22) contre la paroi interne (12a) du stator cylindrique (12) pour transporter, de ce fait, un liquide le long du tubage flexible (22) ;
dans laquelle la bague pouvant se déformer radialement (28 ; 56) comprend une ancre annulaire (30) ;
dans laquelle le côté entrée (24) et le côté sortie (26) du tubage flexible (22) sont disposés côte à côte de telle sorte que le tubage flexible (22) s'étende dans une direction sensiblement radiale vers l'extérieur à l'opposé du stator cylindrique (12) ;
**caractérisée en ce que** l'ancre annulaire (30) fait saillie radialement vers l'extérieur depuis la bague pouvant se déformer radialement (28 ; 56) et est située entre le côté entrée (24) et le côté sortie (26), et est saisie par ceux-ci, du tubage flexible (22) pour empêcher la rotation de la bague pouvant se déformer radialement (28 ; 56) pendant la rotation du rotor pouvant être entraîné (14 ; 52).

2. Pompe selon la revendication 1, dans laquelle l'ancre annulaire (30) comprend un doigt (32) faisant saillie radialement vers l'extérieur depuis la bague pouvant se déformer radialement (28, 56).

3. Pompe selon la revendication 1 ou la revendication 2, dans laquelle la bague pouvant se déformer radialement (28, 56) présente une profondeur axiale qui est plus importante qu'un diamètre externe du tubage flexible (22).

4. Pompe selon l'une quelconque des revendications précédentes, dans laquelle la bague pouvant se déformer radialement (28, 56) comprend une pluralité de saillies radiales espacées circonférentiellement (36, 58) sur un premier bord axial (28a, 56a) qui font saillie dans une direction radialement vers l'extérieur vers la paroi interne (12a) du stator cylindrique (12).

5. Pompe selon la revendication 4, dans laquelle le rotor (14, 52) comprend une bride circulaire (14a, 52a) qui retient axialement le tubage flexible (22) et la bague pouvant se déformer radialement (28, 56) dans le stator cylindrique (12) et dans laquelle la bague pouvant se déformer radialement (56) est agencée dans le stator (12) avec les saillies radiales (58) en contact avec la bride circulaire (52a).

6. Pompe selon l'une quelconque des revendications précédentes, dans laquelle la bague pouvant se déformer radialement (28, 56) comprend un agencement de positionnement (34, 60), sur un second bord axial (28b, 56), qui s'étend depuis l'ancre annulaire (30) sur le côté entrée (24) et le côté sortie (26) du tubage flexible (22).

7. Pompe selon la revendication 6, dans laquelle l'agencement de positionnement (34, 60) comprend une bride de positionnement (38) d'une paire de saillies de positionnement s'étendant à l'opposé (62).

8. Pompe selon l'une quelconque des revendications précédentes, dans laquelle le rotor (14, 52) comprend une pluralité d'éléments de pression (15) qui sont équidistants autour de la circonférence du rotor (14, 52).

9. Pompe selon l'une quelconque des revendications précédentes, dans laquelle le rotor (14, 52) comprend un axe (18) et l'élément de pression ou chaque élément de pression (15) est formé d'un seul tenant avec l'axe (18).

10. Pompe selon l'une quelconque des revendications précédentes, dans laquelle l'élément de pression ou chaque élément de pression (15) est un lobe (16, 54).

11. Pompe selon la revendication 10, dans laquelle le lobe ou chaque lobe (54) comporte une surface de pression en forme d'arc (54a) qui est agencée pour comprimer de manière progressive le tubage flexible (22) contre la paroi interne (12a) du stator cylindrique (12) pendant la rotation du rotor (52).

12. Pompe selon la revendication 11, dans laquelle le lobe ou chaque lobe (54) comporte un sommet (54b) au niveau duquel la surface de pression en forme d'arc (54a) se termine, le sommet (54b) étant agencé pour comprimer complètement le tubage flexible (22) contre la paroi interne (12a) du stator cylindrique (12).

13. Pompe selon la revendication 11 ou la revendication 12, dans laquelle le rotor (52) comprend deux desdits lobes (54) à des emplacements diamétralement opposés.

14. Pompe selon l'une quelconque des revendications précédentes, dans laquelle le rotor (14, 52) peut être mis en prise par un entraînement rotatif externe.

15. Procédé pour assembler une pompe péristaltique (10 ; 50) selon l'une quelconque des revendications précédentes, le procédé consistant :
à placer le tubage flexible (22) de manière circonférentielle autour de la bague pouvant se déformer radialement (28 ; 56) et en contact avec celle-ci, le côté entrée (24) et le côté sortie (26) du tubage flexible (22) étant agencés côte à côte sur l'un ou l'autre côté de l'ancre annulaire (30) ; grâce à quoi l'ancre annulaire (30) est saisie par le côté entrée (24) et le côté sortie (26) du tubage flexible (22) pour empêcher la rotation de la bague pouvant se déformer radialement (28 ; 56) pendant la rotation du rotor (14 ; 52),
à positionner le tubage flexible (22) et la bague pouvant se déformer radialement (28 ; 56) dans le stator cylindrique (12), le tubage flexible (22) étant agencé contre la paroi interne (12a) du stator cylindrique (12) ; et
à ajuster le rotor (14; 52) dans le stator cylindrique (12) en faisant tourner en même temps le rotor (14, 52) et en pressant le rotor (14 ; 52) au centre de la bague pouvant se déformer radialement (28 ; 56).
